# EUROPEAN PATENT APPLICATION

(11) **EP 3 892 203 A1**
(43) Date of publication of application: **13.10.2021**
(21) Application number: 21157502.2
(22) Date of filing: 14.03.2014
(51) Int. Cl.: A61B 10/00, A61B 10/02

(54) **APPARATUSES FOR AMNIOTIC FLUID COLLECTION**

(30) Priority: 15.03.2013 US 201361801256 P
(62) Divisional of application: 19210935.3
(71) Applicant: Amniotics AB, 223 63 Lund (SE)
(72) Inventor: Larsson, Marcus Kare Torleif, 237 34 Bjärred (SE); Herbst, Andreas Nils Walter, 237 34 Bjärred (SE); Woods, Niels-Bjarne Roland, 244 60 Furulund (SE)
(74) Representative: Invent Horizon IP

(57) **Abstract**

Disclosed herein are methods and apparatuses for the safe, high-yield collection of sterile amniotic fluid. The apparatus are configured to allow sufficient flexibility for the device to gain access to all areas of the amniotic cavity, to allow sufficient stiffness for the amniotic fluid collection device to puncture the amniotic and chorionic membranes, yet allow sufficient suppleness to pose no significant risk of maternal or fetal harm. Further disclosed are cells isolated from the full-term amniotic fluid, and methods for using, reprogramming, and differentiating these cells.

## Description

### Technical Field

In one embodiment, the invention described herein relates to obtaining biological material, including amniotic fluid and cells, including at birth, and in some aspects relates to safe, high-yield collection of sterile amniotic fluid and devices and methods for the same. In another embodiment, the invention relates to cells and cultures of cells derived from amniotic fluid, such as fetal or infant cells, and methods for isolating, expanding, reprogramming, differentiating, and storing the cells, and uses of the cells in therapeutics, treatment, disease prevention, drug discovery, personalized medicine, regenerative medicine, tissue generation, and universal donor banks, and related methods and compositions.

### Background

Methods for isolating cells, cell reprogramming, generating pluripotent and multipotent cells, and tissue, organ, and stem cell therapies, are needed for a variety of therapeutic applications, including personalized and regenerative medicine. A variety of human stem cells and other cell types are known, including embryonic stem cells, isolated during early embryonic development, and somatic stem cells such as mesenchymal or adult stem cells. Some non-stem cells can be reprogrammed into more primitive stages.

Amniotic fluid is the aqueous medium that surrounds, protects, and aids in the fetal development, for example, by providing a mechanical barrier, providing growth factors, and aiding lung development by filling developing air spaces to define what will become permanent air spaces. Amniotic fluid contains numerous molecular components, including lung surfactant, cytokines, growth factors, vernix, and propagating survival signals.

Methods for isolating fetal cells from amniotic fluid are described in U.S. Patent No. 7,596,385, U.S. Patent Publication Numbers US 2005/0054093 and US 2005/0042595, all to Haas, and in U.S. Patent Publication Number US 2005/0124003 and International Patent Application No. WO 03/042405, to Atala et al. Methods and apparatuses for collecting amniotic fluid include amniocentesis, performed during the first or second trimester of pregnancy, and the method described by M. Hallman et al., Isolation of Human Surfactant from Amniotic Fluid and a Pilot Study of its Efficacy in Respiratory Distress Syndrome, Pediatrics 1983, 71(4): 473-482, which utilizes a standard operating-room active suction device.

Available approaches and apparatuses for collecting, extracting, and isolating biological components, including cellular material, amniotic fluid, and cells found therein, have not been entirely satisfactory, for example, in their safety, avoidance of contamination (e.g., air contamination) of collected material, cell yield, efficiency, and/or ability to avoid destruction of components.

Methods for isolating stem cells and/or generating multipotent and/or pluripotent stem cells from more differentiated cells are needed, for example, for cell based therapeutic applications, including regenerative medicine, and personalized medicine (individually optimized therapy). Cell sources and isolation methods are needed in this regard. There also is a need for source material cells that can be harvested conveniently from a large number of donors, for example, for isolation of cells in high numbers and that are capable of being manipulated. Provided are embodiments that address such needs.

For example, there is a need for methods and apparatuses for safe, high-yield collection of sterile amniotic fluid, with minimal destruction of amniotic fluid components, for retrieval, propagation, and differentiation of components, including cells, present in the amniotic fluid at various stages of pregnancy, and for safe and reliable methods for extracting and isolating cellular material with the potential for reprogramming generating pluripotent and multipotent cells, and, without compromising existing sources of therapeutically relevant cells.

Likewise, there is a need for cells that are abundant and capable of manipulation, reprogramming, and differentiation, for use in regenerative medicine, treatment and disease prevention, personalized medicine, tissue generation, and universal donor banks are needed. Among the provided embodiments are methods, apparatuses, cells, and compositions that address these needs.

### Summary

Among the provided embodiments are apparatuses and devices for collection of amniotic fluid, *e.g.,* late or full-term amniotic fluid, and for isolation, expansion, reprogramming, and differentiation of cells, *e.g.,* fetal or infant cells, derived from amniotic fluid, cells and compositions obtained by such methods, and methods for using the cells and compositions, such as in treatment and regenerative and personalized medicine.

An amniotic fluid collection device in accordance with an exemplary embodiment of the present invention includes a collection chamber that receives amniotic fluid from an extraction tube, wherein the extraction tube is fitted with an inlet configured to be inserted into an amniotic sac. The extraction tube and inlet are configured to allow sufficient flexibility for the device to gain access to all areas of the amniotic cavity, to allow sufficient stiffness for the amniotic fluid collection device to puncture the amniotic and chorionic membranes, yet allow sufficient suppleness to pose no significant risk of maternal or fetal harm. In this way, the present invention allows for a safe, high-yield collection of sterile amniotic fluid.

An amniotic fluid collection device in accordance with another exemplary embodiment of the present invention comprises an inlet which includes a main inlet on the distal portion of the inlet and at least one auxiliary inlet on a side portion of the inlet. In this way, the present invention avoids blockages that may cause delay, thereby reducing the time necessary for collection and, consequently, reducing the risk of maternal and fetal harm. In addition, this embodiment eliminates the risk of fetal skin-bruising that could result when the main inlet attaches to the fetal surface.

An amniotic fluid collection device in accordance with yet another exemplary embodiment of the present invention comprises an inlet configured to achieve an impervious seal between the extraction tube and the amniotic membranes. In this way, the present invention prevents egress of uncollected amniotic fluid from the amniotic sac and ingress of contaminants to the amniotic sac, thereby increasing yield and maintaining sterility of the amniotic fluid. Additionally, the impervious seal of this exemplary embodiment facilitates a siphon for transferring the amniotic fluid, which obviates the need for an external suction source, thereby further reducing the risk of amniotic fluid contamination.

In one embodiment, provided herein is an amniotic fluid collection device, comprising: a collection chamber; a collection chamber outlet valve coupled to the collection chamber; a collection chamber inlet valve coupled to the collection chamber; a proximal fluid-extraction tube coupled to the collection chamber inlet valve, the proximal fluid-extraction tube comprising a medical grade material; and a distal fluid-extraction tube coupled to the proximal fluid-extraction tube, the distal fluid extraction tube comprising a transfer portion and an inlet, wherein the inlet comprises a main inlet, a penetrating tip, and one or more lateral inlets.

In one embodiment, provided herein is an isolated fetal or infant cell, comprising surface expression of a marker selected from the group consisting of CD73 and CD90, wherein the surface of the cell is negative for CD105 expression; or the surface of the cell expresses CD34. In one aspect, the isolated fetal or infant cell comprises surface expression of CD73 and CD90. In one aspect, in any of the foregoing embodiments, the isolated fetal or infant cell surface can be negative for CD105 expression and express CD34. In any of the foregoing embodiments, the isolated fetal or infant cell can express the transcription factor Oct-4. In any of the foregoing embodiments, the isolated fetal or infant cell surface may be negative for CD45 surface expression. In any of the foregoing embodiments, the isolated fetal or infant cell may be adherent. In any of the foregoing embodiments, the isolated fetal or infant cell can have a fibroblastic morphology. In any of the foregoing embodiments, the isolated fetal or infant cell may be pluripotent or multipotent. In one aspect, in any of the foregoing embodiments, the isolated fetal or infant cell is capable of growing in continuous culture for at least 50 days; or the cell is capable of proliferating in culture for at least 30 population doublings; or the cell is capable of expanding at least 1,000,000 fold in continuous culture.

In one aspect, provided herein is a composition, comprising a fetal or infant cell of any of the foregoing embodiments or any combinations thereof.

In one embodiment, provided herein is a composition, comprising a plurality of fetal or infant cells, wherein at least 10 % of the cells have surface expression of CD90, CD73, and CD34 and less than 5 % of the cells have surface expression of CD105. In one aspect, at least 50 % of the cells express the transcription factor Oct-4. In one embodiment, less than 5 % of the cells have CD45 surface expression. In one aspect, at least 50 % of the cells are adherent. In another aspect, at least 50 % of the cells have a fibroblastic morphology. In another aspect, at least 50 % of the cells are pluripotent or multipotent. In one embodiment, at least 50 % of the cells are capable of growing in continuous culture for at least 50 days; or the plurality of cells is capable of proliferating in culture for at least 30 population doublings; or the plurality of cells is capable of expanding at least 1,000,000 fold in continuous culture. In any of the foregoing composition embodiments and any combination thereof, the composition further comprises amniotic fluid. In one aspect, the cells are present at a concentration of at least 1 million cells per liter.

In one embodiment, provided herein is a method for isolating fetal or infant cells, comprising: (a) obtaining amniotic fluid at a gestational stage of greater than 21, 22, 23, 24, 25, 26, 27, 28, 29, 30, 31, 32, 33, 34, 35, 36, 37, 38, or 39 weeks; and (b) recovering fetal or infant cells from the amniotic fluid. In one aspect, the obtaining in step (a) comprises collecting amniotic fluid by caesarean section. In any of the foregoing method embodiments, the recovering in step (b) may comprise removing particulate matter from the amniotic fluid. In one aspect, the recovering in step (b) further comprises performing gradient centrifugation. In any of the foregoing method embodiments or any combinations thereof, the method may further comprise culturing the recovered cells under conditions whereby the cells proliferate. In any of the foregoing method embodiments or any combinations thereof, the recovered cells in step (b) may comprise at least 1 million cells per liter of amniotic fluid obtained in step (a). In any of the foregoing method embodiments or any combinations thereof, the amount of amniotic fluid obtained in step (a) may be at least at or about 10 mL, 20 mL, 30 mL, 40 mL, 50 mL, 60 mL, 70 mL, 80 mL, 90 mL, 100 mL, 200 mL, 300 mL, 400 mL, 500 mL, 600 mL, 700 mL, 800 mL, 900 mL, or 1000 mL. In any of the foregoing method embodiments or any combinations thereof, the amniotic fluid can be full-term amniotic fluid. In any of the foregoing method embodiments or any combinations thereof, the fetal or infant cells can comprise the cell of any of the foregoing isolated fetal or infant cell embodiments.

In any of the foregoing method embodiments or any combinations thereof, the fetal or infant cells may comprise a cell that is positive for surface expression of a marker selected from the group consisting of CD73 and CD90, wherein the cell is negative for surface expression of CD105 expression or positive for surface expression of CD34. In one aspect, the cell is positive for surface expression of CD73 and CD90. In one aspect, the cell is negative for surface expression of CD105 and is positive for surface expression of CD34. In one aspect, the cell expresses the transcription factor Oct-4. In another aspect, the cell is negative for CD45 surface expression. In another aspect, the cell is adherent. In one embodiment, the cell has a fibroblastic morphology. In another embodiment, the cell is pluripotent or multipotent. In yet another embodiment, the cell is capable of growing in continuous culture for at least 50 days; or the cell is capable of proliferating in culture for at least 30 population doublings; or the cell is capable of expanding at least 1,000,000 fold in continuous culture.

In any of the foregoing method embodiments or any combinations thereof, the method may further comprise reprogramming the recovered cells, thereby generating multipotent or pluripotent cells. In one aspect, the reprogramming comprises performing iPSC. In one embodiment, the method further comprises differentiating the cells into a desired cell or tissue type. In a further embodiment, the desired cell or tissue type is selected from the group consisting of hematopoietic cells, neuronal cells, endodermal cells, ectodermal cells, and mesodermal cells.

Also provided herein is a method of treatment, comprising administering the composition of any of the foregoing composition embodiments or any combinations thereof to a patient.

In one aspect, disclosed herein is a method for banking cells, comprising: (a) obtaining one or more samples, each containing the cell of any of claims 2-10; (b) storing the sample under conditions to preserve viability of at least some of the cells in the sample; and (c) storing data related to the identity of the subject from which the sample was obtained. In one aspect, the storing comprises treating the sample with one or more cryoprotective agent. In one embodiment, step (a) comprises obtaining a plurality of samples, each obtained from a different subject.

In another aspect, disclosed herein is a cell bank, comprising: (a) a plurality of samples, each containing the cell of any of claims 2-10 or the composition of any of claims 11-20; and (b) a database containing data for individual identification and retrieval of the samples. In one aspect, each of the plurality of samples is derived from an individual subject. In any of the foregoing cell bank embodiments, the data may include information about the identity of the subjects from which individual samples were derived.

In yet another aspect, disclosed herein is a method for collecting amniotic fluid, comprising: (a) inserting an amniotic fluid collection device through an incision in the uterine wall of the subject; (b) penetrating the amniotic membrane of the subject; and (c) collecting amniotic fluid from the amniotic sac of the subject, wherein the subject is a pregnant mother at a stage of at least 30 gestational weeks. In one aspect, the subject is at a full-term stage of gestation. In another aspect, the amniotic fluid collection device is the device of any of the foregoing device embodiments or any combination thereof. In certain aspects, step (b) further comprises penetrating a chronic membrane. In some aspects, the collecting in step (c) includes one or more of: (i) initiating a siphon to transfer the amniotic fluid to a collection chamber of the amniotic fluid collection device by opening an inlet valve of the amniotic fluid collection device; (ii) positioning a collection chamber of the amniotic fluid collection device below an inlet of the amniotic fluid collection device; (iii) coupling a negative pressure source to an outlet of the amniotic fluid collection device to initiate transfer of the amniotic fluid; and (iv) relocating an inlet of the amniotic fluid collection device to retrieve substantially all of the available amniotic fluid. In one embodiment, the method further comprises step (d) removing the amniotic fluid collection device. In some aspects, the incision is made by the amniotic fluid collection device. In some embodiments, the method is performed in less than about 10 minutes, about 9 minutes, about 8 minutes, about 7 minutes, about 6 minutes, about 5 minutes, about 4 minutes, about 3 minutes, about 2 minutes, or about 1 minute.

### Description of the Figures

Figure 1 illustrates an amniotic fluid collection device 100, in accordance with an exemplary embodiment of the present invention.
Figure 2 shows cell surface marker expression of full term amniotic fluid derived cells by FACS analysis. Percentages of cells in the population that are positive for surface expression of the markers are indicated.
Figure 3 illustrates a method 300 of extracting amniotic fluid, in accordance with an exemplary embodiment of the present invention.

### Detailed Description

In the following description of certain embodiments provided here, reference is made to the accompanying drawings which form a part hereof, and in which it is shown by way of illustration specific embodiments in which the invention can be practiced. It is to be understood that other embodiments can be used and structural changes can be made without departing from the scope of the invention.

Among the provided embodiments are apparatuses, devices, and methods for collection, isolation, and use of biological materials, including cell and cellular sources, such as those obtained from amniotic fluid, as well as cells and other materials obtained by such methods, and uses thereof, including therapeutic and analytic uses, for example, in regenerative medicine, including personalized and universal donor applications.

A variety of human stem cells are being used therapeutically or evaluated for use in clinical trials, including somatic cells, such as mesenchymal stem cells, and hematopoietic stem cells, *e.g.,* for use in neurological and hematological disorders, respectively. Other somatic cells of more differentiated capacities can be reprogrammed into more primitive stages, for example reprogramming of endothelial cells into pluripotent stem cells.

Induced pluripotent stem (iPS) cells have been generated from multiple cell types, including skin and fibroblasts. Masip et al., 2010, Mol Hum Reprod 16(11): 856-868; Takahashi and Yamanaka, 2006, Cell 126(4): 663-676; Yu et al., 2007, Science 318(5858): 1917-1920. Alternative starting materials have been chosen based on: ease of reprogramming, including sufficient proliferation rates, genetic background for disease modeling, ease of harvisitng sufficient numbers of vialbe cells, and genomic integrity. Hanna et al., 2009, Nature 462(7273): 595-601; Park et al., 2008, Cell 134(5): 877-886; Ye et al., 2009, Blood 114(27): 5473-5480; Carette et al., 2010, Blood 115(20): 4039-4042; Kumano, et al., 2012, Blood 119(26):6234-6242; Ikehata et al., 2003, Environ Mol Mutagen 41(4): 280-292; Ikehata et al., 2004, Mutat Res 556(1-2): 11-24; Osanai and Lee, 2011, Med Mol Morphol 44(4): 200-206.

Available methods for generating iPS cells have been associated with certain problems. For example, adult skin fibroblasts, from which a majority of iPS cells are generated. Nonetheless, due to epigenetic memory, the residual epigenetic state of a cell used as a source for iPS generation can influence the differentiation capacity of derived iPS cells. Specifically, iPS cell lines can maintain an epigenetic memory of tissue origin by continuing to express a key set of donor cell genes. Marchetto et al., 2009, PLoS One 4(9): e7076; Ghosh et al., 2010, PLoS One 5(2): e8975; Bar-Nur et al., 2011, Cell Stem Cell 9(1): 17-23. Epigenetic memory can impact differentiation capacity of many cellular sources, which can make certain differentiated cells (such as neural progenitor cells, certain blood lineage cells, fibroblasts, myogenic cells, pancreatic islet beta cells and other pancreatic cells) less desirable for iPS generation. Kim et al., 2010, Nature 467(7313): 285-290; Polo et al., 2010, Nat Biotechnol 28(8): 848-855; Bar-Nur et al., 2011, Cell Stem Cell 9(1): 17-23; Marchetto et al., 2009, PLoS One 4(9): e7076; Ghosh et al., 2010, PLoS One 5(2): e8975.

Thus, more primitive cells with multipotent differentiation capacities in some contexts are a more appropriate starting material for iPS cell generation, with broad differentiation capacities.

In some contexts, naive cells that have had minimal contact with the environment, contain undamaged genome(s), and/or have the ability to proliferate and/or expand in an *in vitro* culture system, are desired for cell reprogramming, e.g., for tissue, organ, and/or stem cell based therapies. Certain cellular material derived from a term or near term fetus or newborn child would fulfill those characteristics. At birth, cellular sources, e.g., amniotic fluid cells, contain cells having such characteristics, but typically are discarded.

Amniotic fluid contains molecular components, including viable cellular material, shed from the fetus and amniotic membrane into the fluid throughout development. Such shed cells represent the developmental state of the fetus. With increasing bio-surface of the fetus (e.g. lung development), a higher number of shed cells is expected. The turnover of amniotic fluid is high, up to several times per 24 hours. Thus any collected cells are not in suspension in this medium for an extended period of time. The cellular content is considered mainly of fetal origin.

Available methods for harvesting such cellular sources available at birth have not been entirely satisfactory, for example, relating to safety concerns for mother and child. For example, the presence of contaminants, such as maternal blood an air, in amniotic fluid obtained by available methods can compromise sterility, resulting, for example, in damage and structural alterations to molecular components of the fluid, introduction of air-borne pathogens, and interference with the hypoxic state of the cells in the amniotic fluid, causing inactivation of proteins. Lack of efficiency and prolonged procedures can cause maternal or fetal harm. For example, amniocentesis, generally performed during the first or second trimester, yields limited quantities (generally approximately 2 mL) of amniotic fluid and requires the use of a sharp syringe (*e.g.*, by ultrasonic guidance) inserted through the maternal abdomen into the amniotic cavity, which carries a significant risk for fetal harm or even demise (*e.g.* 1-10 %). The method described by M. Hallman et al., Isolation of Human Surfactant from Amniotic Fluid and a Pilot Study of its Efficacy in Respiratory Distress Syndrome, Pediatrics 1983, 71(4): 473-482, which utilizes a standard operating-room active suction device, introduces contaminants into the system through an incision made in the uterine wall, through which the suction device is inserted into the amniotic sac.

Alternatives to embryonic stem cell isolation, such as use of adult mesenchymal stem cells (MSCs) and reversion of differentiated cells into pluripotent and multipotent cells (such as by induction of pluripotent stem cells (iPSC)) can avoid disadvantages such as political, moral and ethical issues. Such methods can also avoid safety concerns associated with available approaches to amniotic fluid collection. Nonetheless, use of adult cells carries the risk of reduced genetic quality, such as from exposure to mutagens such as UV radiation, smoke, pesticides, and microbes, which can cause mutations and lead to cancerous or undesirable behavior in tissue generation and treatment.

Provided are compositions, methods, and apparatuses that address these difficulties.

In certain embodiments, provided are devices, apparatuses, and methods for the collection of amniotic fluid, for example, full or near term amniotic fluid, for example, for retrieval of medically valuable components in the amniotic fluid material. In some aspects, such collection occurs during a cesarean section. In some aspects, such embodiments are useful for sterile harvest of amniotic fluid, such as fluid that is free of maternal blood, air, and/or other contaminants, for safe and efficient collection of amniotic fluid, for example, at later gestational stages, *e.g*., full-term. In some aspects, such embodiments involve no or minimal destruction of amniotic fluid components, pose no substantial risk of maternal or fetal harm, and/or yield a high-percentage of available fluid. In some embodiments, such devices and apparatuses and methods have properties allowing routine use at cesarean delivery, such as ease of use and minimal interference with the operative procedure, for example, by use of single use sterile units, minimizing contacts with the ambient air and maximizing sterility. Thus, among the provided embodiments are methods and apparatuses for safe, sterile, high-yield collection of amniotic fluid with minimal destruction of components.

Also provided are methods for harvest, isolation, and expansion of material obtained from such devices, apparatuses, and methods, including cells and cellular fractions, and uses thereof, including medical uses, such as uses in treatment and regenerative methods. Also provided is material obtained by use of such methods, devices, and apparatuses, including cells and cellular fractions from amniotic fluid and compositions containing the same.

Also provided are methods for expanding, reprogramming, and differentiating such cells and cellular fractions, and methods for use of the same, e.g., for cellular and tissue generation, regenerative and personal medicine, disease treatment, and other processes.

### A. Definitions

Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. Many of the techniques and procedures described or referenced herein are well understood and commonly employed using conventional methodology by those skilled in the art.

Throughout this disclosure, various aspects of this invention are presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. In another example, a description of a range in weeks also includes disclosure of the days between the week endpoints. This applies regardless of the breadth of the range.

As used herein, the term "amniotic fluid" can be understood to refer to the entire fluid and non-fluid components present in the amniotic cavity, such as the liquid, solid, semi-solid, or cellular constituents contained therein, whether in suspension or not.

As used herein, the term "amniotic fluid collection device" refers to any of the exemplary embodiments of the device described herein. It is sometimes referred to as "the device."

As used herein, the term "caesarean section" can be understood to refer to any surgical procedure performed on a pregnant mother involving a laparotomy and a hysterotomy, whether also involving a fetus-delivery or not.

As used herein, the term "cryo-TEM" means cryo-transmission electron micrography.

As used herein, the term "safe" can be understood to refer to any method or apparatus which poses no significant risk of maternal and/or fetal harm.

As used herein, the term "siphon" can be understood to refer to the transfer of liquid between two points, whereby a pressure differential between two locations in the liquid provides the dynamic force for the transfer.

As used herein, the term "vernix" can be understood to refer to the substance secreted by fetal skin *in utero,* which appears in the amniotic fluid as semi-solid whitish lumps or dispersed aggregates of this substance.

As used herein, "full-term" describes a stage during pregnancy that is at least 38 gestational weeks.

As used herein, "post-term" describes a stage of pregnancy that is greater than 41 weeks.

As used herein, "isolated," when used to describe a cell or cells, refers to a cell or cells that have been separated from their natural environment, including by separation from the subject from which the cell is derived, *e.g.,* a patient, and/or by separation from one or more other components of the natural environment, such as amniotic fluid, debris, vernix, tissue, tissue aggregates, and other cells.

As used herein, "fetal" is used to describe the property of a cell or other material derived from a developing mammal, such as a human, after the embryonic stage and before birth. As used herein, "infant" is used to describe the property of a cell or other material derived from a newborn or young mammal, from birth to one year of age, including premature infants and newborns. The cells and compositions provided herein typically are isolated from late-stage pregnancy or full-term amniotic fluid, and thus generally include fetal cells and early-stage infant cells, such as newborn or neonate cells, for example, from an infant no more than a day in age.

As used herein, "pluripotent" refers to the ability of a cell to differentiate into cell types of any of the three germ layers, endoderm, mesoderm, and ectoderm. "Multipotent" refers to the ability of a cell to differentiate into cells of a number of multiple, but a limited number of lineages.

### B. Amniotic Fluid Collection Device

Provided herein are methods and apparatus designed to retrieve amniotic fluid.

An amniotic fluid collection device in accordance with an exemplary embodiment of the present invention includes a collection chamber that receives amniotic fluid from an extraction tube, wherein the extraction tube is fitted with an inlet configured to be inserted into an amniotic sac. The extraction tube and inlet are configured to allow sufficient flexibility for the device to gain access to all areas of the amniotic cavity, to allow sufficient stiffness for the amniotic fluid collection device to puncture the amniotic and chorionic membranes, yet allow sufficient suppleness to pose no significant risk of maternal or fetal harm. In this way, the present invention allows for a safe, high-yield collection of sterile amniotic fluid.

An amniotic fluid collection device in accordance with another exemplary embodiment of the present invention comprises an inlet which includes a main inlet on the distal portion of the inlet and at least one auxiliary inlet on a side portion of the inlet. In this way, the present invention avoids blockages that may cause delay, thereby reducing the time necessary for collection and, consequently, reducing the risk of maternal and fetal harm. In addition, this embodiment eliminates the risk of fetal skin-bruising that could result when the main inlet attaches to the fetal surface.

An amniotic fluid collection device in accordance with yet another exemplary embodiment of the present invention comprises an inlet configured to achieve a substantially impervious seal between the extraction tube and the amniotic membranes. In this way, the device prevents egress of uncollected amniotic fluid from the amniotic sac and ingress of contaminants to the amniotic sac, thereby increasing yield and maintaining sterility of the amniotic fluid. Additionally, the substantially impervious seal of this exemplary embodiment facilitates a siphon for transferring the amniotic fluid, which obviates the need for an external suction source, thereby further reducing the risk of amniotic fluid contamination.

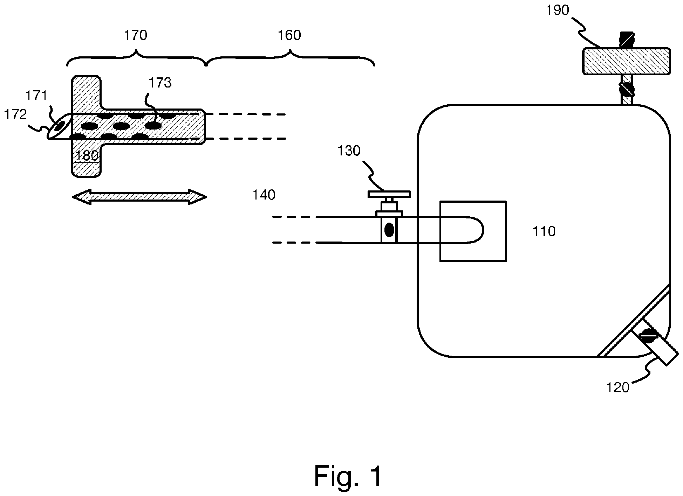

Figure 1 illustrates an amniotic fluid collection device 100 in accordance with an exemplary embodiment of the present invention. Amniotic fluid collection device 100 includes a collection chamber 110, a collection chamber outlet valve 120, a collection chamber inlet valve 130, a proximal fluid extraction tube section 140, and a distal fluid extraction tube section 150. Distal fluid extraction tube section 150 includes transfer portion 160 and inlet 170. Inlet 170 includes main inlet 171, penetrating tip 172, and one or more lateral inlets 173.

Inlet 170 is inserted into an amniotic sac and amniotic fluid is transferred from the sac to the collection chamber 110 via the fluid extraction tube, comprising proximal fluid extraction tube section 140 and distal fluid extraction tube section 150. The proximal fluid extraction tube section 140 may comprise medical grade tubing of a desired length. When the procedure is completed, the collection chamber inlet valve 130 may be closed and the fluid extraction tube 140 and 150 removed. Collection chamber outlet valve 120 may be opened to drain collected amniotic fluid from collection chamber 110.

Distal fluid extraction section 150 is configured to be inserted into an amniotic sac. In one embodiment, distal fluid extraction tube section 150 is configured to achieve a substantially impervious seal with the amniotic sac membranes when inserted. This substantially impervious seal provides many salient functional advantages. First, the impervious seal maintains the pressure differential between the interior and exterior of the amniotic sac. This pressure differential permits the slightly higher ambient pressure of the amniotic fluid to initiate siphoning of the amniotic fluid from the sac to the collection chamber 110. In one embodiment, the collection chamber 110 is positioned below the inlet 170, whereby gravitational force facilitates the transfer until substantially all of the amniotic fluid material is collected. An optional negative pressure pump may be added to collection chamber outlet valve 120 to initiate siphoning.

A second exemplary advantage of the substantially impervious seal is prevention of contaminant ingress to the amniotic sac.

A third advantage is that the siphon obviates the need for a vacuum pump, which can accelerate the desterilization of the amniotic fluid.

A fourth advantage is that the seal also prevents egress of uncollected amniotic fluid from the amniotic sac, thereby increasing yield and improving visibility and physical access for fetal delivery.

In one example, the material of distal fluid extraction section 150 is selected such that a substantially impervious seal is created between the distal fluid extraction section 150 and one or more amniotic sac membranes. In certain embodiments, the distal fluid extraction section 150 is manufactured from polyvinyl chloride ("PVC"), poly(ethylene terephthalate), polyurethane, or poly(propylene carbonate), or any suitable combination thereof. In some embodiments, softeners may be added as needed for a required stiffness. Without being bound by any theory, any suitable moldable polymer plastics may be used for manufacturing the distal fluid extraction section 150. In other embodiments, nylon, silicone and polypropylene are used. Without being bound by any theory, any suitable material can be used for the piercing end of the distal fluid extraction section 150, as long as it is strong enough to pierce the amniotic sac, yet pliable enough to pose a non-substantial risk of fetal harm. The cutting angle and the material properties contribute to the functions of the distal fluid extraction section 150. In some embodiments, the cutting angle is between about 0 degree and about 90 degrees. In preferred embodiments, the cutting angle is between about 25 degrees and about 75 degrees. In certain embodiments, concave or convex forms of the angle may be employed depending on the catheter material. In further embodiments, a ring of soft material is added to the outside of the catheter tubing to facilitate the formation of the substantially impervious seal.

In one embodiment, the dimensions of distal fluid extraction section 150 and proximal fluid extraction device 140 are sufficiently large to obtain a rapid fluid extraction, yet not so large as to prevent siphon initiation. The French scale or French gauge system, abbreviated as Fr, is commonly used to measure the size of a catheter. A catheter of 1 French has a diameter of ⅓ mm and therefore the diameter of a round catheter in millimeters can be determined by dividing the French size by 3: D (mm) = Fr/3. In some embodiments, a French gauge catheter in the range of about 13 to about 34 (about 4.3 mm to about 11.3 mm for the external diameter) is used in clinical settings. The inner diameter is dependent on the material used. Without being bound by any theory, any inner diameter suitable for maintaining the stiffness and maneuverability of the material may be used.

In one embodiment, the material of distal fluid extraction section 150 is selected to provide sufficient flexibility to allow the user to maneuver the inlet 170 into pockets of fluid surrounding the fetus as the fluid drains during collection. In this way, the yield of amniotic fluid is further increased. In certain embodiments, the distal fluid extraction section 150 is manufactured from polyvinyl chloride ("PVC"), poly(ethylene terephthalate), polyurethane, or poly(propylene carbonate), or any suitable combination thereof. In some embodiments, softeners may be added as needed for a required stiffness. Without being bound by any theory, any suitable moldable polymer plastics may be used for manufacturing the distal fluid extraction section 150. In other embodiments, nylon, silicone and polypropylene are used.

In one embodiment, inlet 170 serves as both the incision instrument and the collection system inlet. In one embodiment, inlet 170 is configured to permit penetration of the amniotic sac, while also minimizing fetal injury. In one aspect, similarly to distal fluid extraction section 150, the dimensions and material of inlet 170 are selected so that the distal fluid extraction section 150 is strong enough to pierce the amniotic sac, yet pliable enough to pose a non-substantial risk of fetal harm. In one embodiment, the penetrating tip 171 is angled at about 0 degree to about 90 degrees. In preferred embodiments, the penetrating tip 171 is angled at about 25 degrees to about 75 degrees. In certain embodiments, concave or convex forms of the angle may be employed depending on the catheter material. In some embodiments, the penetrating tip 171 is angled at about 25 degrees, about 30 degrees, about 35 degrees, about 40 degrees, about 45 degrees, about 50 degrees, about 55 degrees, about 60 degrees, about 65 degrees, about 70 degrees, or about 75 degrees. In one embodiment, the penetrating tip 171 is angled at less than 25 degrees. In another embodiment, the penetrating tip 171 is angled at more than 75 degrees. In one embodiment, the penetrating tip 171 is angled at 45 degrees. In some embodiments, the inlet 170 is constructed of polyvinyl chloride ("PVC"), poly(ethylene terephthalate), polyurethane, or poly(propylene carbonate), or any suitable combination thereof. In some embodiments, softeners may be added as needed for a required stiffness. Without being bound by any theory, any suitable moldable polymer plastics may be used for manufacturing the inlet 170. In other embodiments, nylon, silicone and polypropylene are used. In one embodiment, the inner diameter of the inlet 170 is 10 mm. In one embodiment, the inner diameter of the inlet 170 is about 10 mm. In certain aspects, the inner diameter of the inlet 170 is about 8.0 mm, about 8.1 mm, about 8.2 mm, about 8.3 mm, about 8.4 mm, about 8.5 mm, about 8.6 mm, about 8.7 mm, about 8.8 mm, about 8.9 mm, about 9.0 mm, about 9.1 mm, about 9.2 mm, about 9.3 mm, about 9.4 mm, about 9.5 mm, about 9.6 mm, about 9.7 mm, about 9.8 mm, or about 9.9 mm. In certain aspects, the inner diameter of the inlet 170 is about 10.1 mm, about 10.2 mm, about 10.3 mm, about 10.4 mm, about 10.5 mm, about 10.6 mm, about 10.7 mm, about 10.8 mm, about 10.9 mm, about 11.0 mm, about 11.1 mm, about 11.2 mm, about 11.3 mm, about 11.4 mm, about 11.5 mm, about 11.6 mm, about 11.7 mm, about 11.8 mm, about 11.9 mm, or about 12.0 mm. In one embodiment, the outer diameter of the inlet 170 is 12.5 mm. In one embodiment, the outer diameter of the inlet 170 is about 12.5 mm. In certain aspects, the outer diameter of the inlet 170 is about 10.5 mm, about 10.6 mm, about 10.7 mm, about 10.8 mm, about 10.9 mm, about 11.0 mm, about 11.1 mm, about 11.2 mm, about 11.3 mm, about 11.4 mm, about 11.5 mm, about 11.6 mm, about 11.7 mm, about 11.8 mm, about 11.9 mm, about 12.0 mm, about 12.1 mm, about 12.2 mm, about 12.3 mm, or about 12.4 mm. In certain aspects, the outer diameter of the inlet 170 is about 12.6 mm, about 12.7 mm, about 12.8 mm, about 12.9 mm, about 13.0 mm, about 13.1 mm, about 13.2 mm, about 13.3 mm, about 13.4 mm, about 13.5 mm, about 13.6 mm, about 13.7 mm, about 13.8 mm, about 13.9 mm, about 14.0 mm, about 14.1 mm, about 14.2 mm, about 14.3 mm, about 14.4 mm, or about 14.5 mm.

Various plastics and dimensions have been empirically determined by testing on amniotic membranes discarded after delivery, coupled with tests on areas of human skin with low keratinisation, i.e., areas with a high susceptibility to penetration or tissue damage, to mimic the effect on the fetus.

In some embodiments, inlet 170 includes one or more lateral inlets 173. Lateral inlets 173 are provided to prevent blockages that may occur when the main inlet 171 comes into contact with any surface within the amniotic cavity, such as amniotic membranes, chorionic membranes, or the fetus. Further, the lateral inlets 173 provide a pressure relief system should the main inlet 171 attach to the amniotic membranes, chorionic membranes, or external fetus surfaces. Use of amniotic fluid collection device without such a pressure relief system can require discontinuation of the suction system or the physical manipulation of the device from the fetus, each of which can result in interrupted collection and high risk of fetal harm, such as long-term skin-bruising. In one aspect of this embodiment, the inlet 170 should be sized to ensure the auxiliary inlets 173 are constantly submerged in the amniotic sac during operation of the amniotic fluid collection device 100; in this aspect, if all auxiliary inlets 173 are not submerged, air will be introduced into the device, obliterating fluid flow and contaminating the yield. In one embodiment, the inlet 170 is from about 1.5 cm to about 15.0 cm in length. In one embodiment, the inlet 170 is less than 10cm in length. In some embodiments, the inlet 170 is about 1.5 cm, about 2.0 cm, about 2.5 cm, about 3.0 cm, about 3.5 cm, about 4.0 cm, about 4.5 cm, about 5.0 cm, about 5.5 cm, about 6.0 cm, about 7.5 cm, about 8.0 cm, about 8.5 cm, about 9.0 cm, about 9.5 cm, about 10.0 cm, about 10.5 cm, about 11.0 cm, about 11.5 cm, about 12.0 cm, about 12.5 cm, about 13.0 cm, about 13.5 cm, about 14.0 cm, about 14.5 cm, or about 15.0 cm. In some embodiments, the inlet 170 is more than 15.0 cm in length.

The amniotic fluid collection device 100 described herein is configured to minimize contamination of the collected amniotic fluid. In one embodiment, a further reduction in contamination is achieved by pre-filing the device with an inert or medically relevant gas, such as, but not limited to, Nitrogen, Xenon, or Nitric Oxide, to name a few. As such, in one embodiment, the device tip may be fitted with a removable seal (not shown) that covers the inlet holes of the tip for removal just prior to device insertion.

In some embodiments, the dimensions of main inlet 171 are selected to avoid clogging by vernix, blood clots, or other materials in the fluid collection device 100. In amniotic fluid collection devices that rely upon external suction, the suction pressure may be increased to overcome the clogging, which can increase the risk of introducing air and other contaminants into the system. In an embodiment of the present invention, clogging may be prevented by configuring the inlet 170 so that the inner diameter of main inlet 171 is no wider than the inner diameter of the proximal fluid extraction tube section 140 and/or the distal fluid extraction tube section 150. Further, in other embodiments, the maximum diameter of the lateral inlets 173 located on the circumference of the inlet 170 may be narrower than the internal diameter of the inlet 170.

Although, for illustrative purposes, the many structures and functions of the exemplary embodiments above have been described with reference to a single device, it should be readily appreciated that any of these structures and functions may be utilized alone or in any combination without deviating from the scope of the present invention.

The amniotic fluid collection devices described above may be pre-packaged into a sterile, ready-to-use configuration. Further, an external removable cover may be used to seal the inlet(s), thereby maintaining the sterility and containing the medically relevant gases, if used, within the tubing until the tip is fully inserted into the amniotic cavity. In some embodiments, this removable cover is configured to perform one or more of the functions of the inlet 170, such as include a penetrating tip and possessing sufficient stiffness to penetrate the amniotic sac.

### C. Collection of amniotic fluid and isolation, culture, reprogramming, and differentiation of cells

Provided are methods for collecting amniotic fluid and isolating cells from the amniotic fluid. In some embodiments, the methods are performed by collecting amniotic fluid, e.g., using a device according to the present invention. In other embodiments, the methods include obtaining fetal and/or infant cells from the amniotic fluid, such as cells shed from fetal epithelial surfaces in topological connection with the amniotic fluid.

### Collection of amniotic fluid at late stage of pregnancy or full-term

Typically, the amniotic fluid is collected at a late stage of pregnancy or at full-term, such as a stage later than the second trimester or later than 21 gestational weeks, for example, at least 22, 23, 24, 25, 26, 27, 28, 29, 30, 31, 32, 33, 34, 35, 36, 37, 38, 39, 40, or 41 gestational weeks, during the third trimester, or at full-term (at least 38 gestational weeks), or post-term (greater than 41 gestational weeks). In one example, the amniotic fluid is isolated during caesarean delivery, for example, full-term caesarean delivery, for example, a caesarean delivery be performed due to an uncomplicated cause, such as breech presentation, previous caesarean delivery, or fear of giving birth vaginally.

Obtaining cells from full- or late-term amniotic fluid can minimize the fetal and maternal risk compared to collection of amniotic fluid at an earlier stages of pregnancy, such as during the first or second trimester, e.g., by amniocentesis. Moreover, the amount of amniotic fluid and number of available cells increase progressively toward full term gestation. Collecting amniotic fluid at or close to full-term can result in high yields. In one aspect, the amniotic fluid is obtained from a single patient at a volume of at least at or about 10 mL, 20 mL, 30 mL, 40 mL, 50 mL, 60 mL, 70 mL, 80 mL, 90 mL, 100 mL, 200 mL, 300 mL, 400 mL, 500 mL, 600 mL, 700 mL, 800 mL, 900 mL, 1000 mL, 1500 mL, or 2000 mL. Isolation of cells from amniotic fluid at late- or full term rather than later in life also can reduce the risk of exposure to mutagens and damage, yielding cells with genomes in naive or more naive states.

Additionally, cells obtained from late- or full-term amniotic fluid have distinct properties. For example, the cellular composition, gene expression profile, and differentiation status of cells shed into the amniotic fluid medium changes over the course of pregnancy, i.e., with fetal age. Maternal hormone expression also varies with gestational stage. For example, lung surfactant production begins at around 30 weeks of gestation, with a maximum quantity reached just prior to term. Because hormones are small enough to pass through the placenta, changes in maternal hormones are reflected in fetal circulation and thus may affect signaling and epigenetic profile of developing fetal cells. For example, the surge in maternal cortisol during the last weeks prior to full term birth induces the maturation of fetal type II pneumocytes, promoting production of lung surfactant (required for airbreathing). This maturation of the type II pneumocytes is maintained throughout the entire life of the individual. Thus, epigenetic alterations in fetal cells are related to gestational stage. Thus, the environment of late-stage, *e.g*., full term, pregnancy imparts unique properties to fetal cells, including maturation status, cell function, and transcriptome profile.

During gestation, amniotic fluid is replaced/recycled every 2 days. This ensures that the cellular content present in the fluid is mostly viable and also has properties associated with the gestational age of the fetus. As such, the cellular material obtained at full term delivery is unique from cellular material from earlier time points. With approximately 200-400 mL of fluid secreted from the lung, and the remaining fluid coming from urine, intra and extracellular spaces, there are potentially many types of cells present in the fluid. Also, an increase in swallowing and kidney function in the third trimester may also affect the cells composition and growth capacity as there is a significant decreases in the osmolarity of the fluid. Modena and Fieni, 2004, Acta Biomed 75 Suppl 1: 11-13.

### Amniotic fluid collection

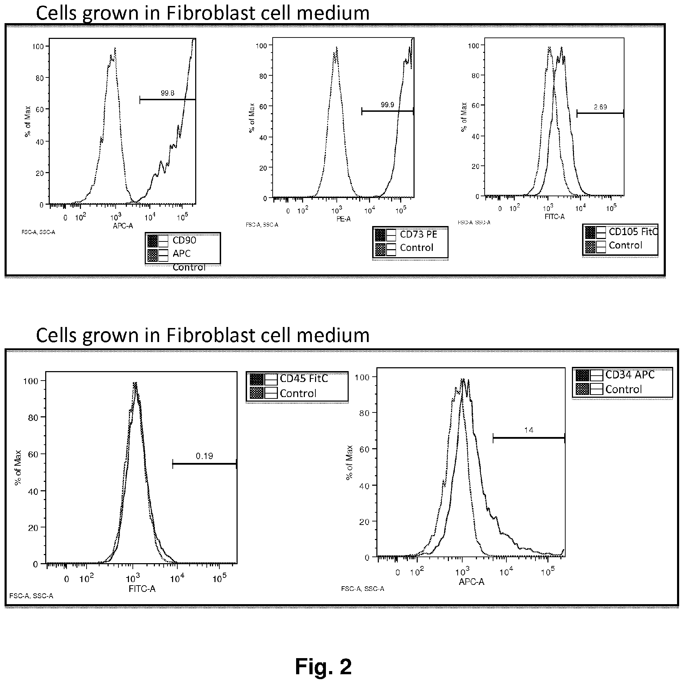

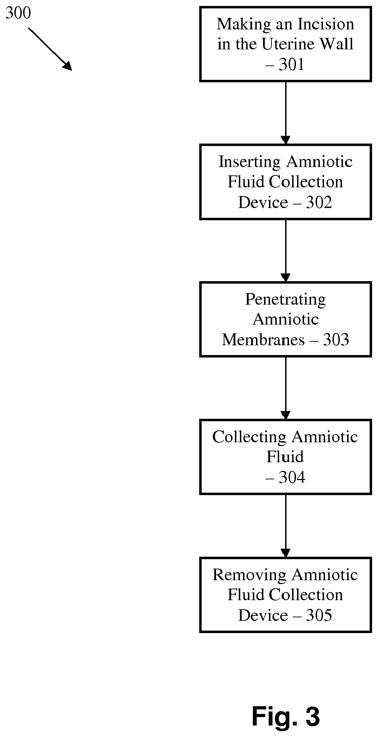

Figure 3 is a block diagram of a method 300 of amniotic fluid collection, according to an exemplary embodiment of the invention. It should be appreciated that method 300 may include any number of additional or alternative tasks. The tasks shown in Figure 3 need not be performed in the illustrated order, and method 300 may be incorporated into a more comprehensive procedure or process having additional functionality not described in detail herein. Method 300 may be implemented using the embodiments illustrated in Figures 1 and 2 and, for illustrative purposes, the following description of method 300 may refer to elements mentioned above in connection with Figures 1 and 2.

As shown in Figure 3, method 300 includes making an incision in the uterine wall 301 of a pregnant mother, for example, during caesarean section. Step 301 may be performed with a standard physician's scalpel. As also shown in Figure 3, method 300 includes inserting an amniotic fluid collection device 302 through the incision in the uterine wall made in Step 301. The amniotic fluid collection device used in Step 302 may comprise any of the embodiments described above with respect to Figure 1 and 2. Method 300 also includes penetrating the amniotic membrane 303 using the amniotic fluid collection device of Step 302. Step 303 may also include penetrating the chorionic membrane. In one aspect, the tip is inserted to a 10 cm depth. In some embodiments, the tip is inserted to a depth of about 3 cm to about 30 cm. In some embodiments, the tip is inserted to a depth of about 4 cm, about 5 cm, about 6 cm, about 7 cm, about 8 cm, about 9 cm, about 10 cm, about 11 cm, about 12 cm, about 13 cm, about 14 cm, about 15 cm, about 16 cm, about 17 cm, about 18 cm, about 19 cm, about 20 cm, about 21 cm, about 22 cm, about 23 cm, about 24 cm, about 25 cm, about 26 cm, about 27 cm, about 28 cm, or about 29 cm.

Method 300 further includes collecting the amniotic fluid 304 from the amniotic sac using the amniotic fluid collection device of Step 302. Step 304 may include initiating a siphon to transfer the amniotic fluid to a collection chamber of the amniotic fluid collection device, such as by opening an inlet valve of the amniotic fluid collection device. Step 304 may also include positioning a collection chamber of the amniotic fluid collection device below an inlet of the amniotic fluid collection device. Step 304 may also include coupling a negative pressure source to an outlet of the amniotic fluid collection device to initiate transfer of the amniotic fluid. Step 304 may include relocating an inlet of the amniotic fluid collection device to retrieve substantially all of the available amniotic fluid.

Finally, method 300 includes removing the amniotic fluid collection device 905 from the amniotic sac. Step 905 may include closing an inlet valve of the amniotic fluid collection device. In one example, no blood is visible in the collected material. Step 905 may also include emptying the collection system for further use/processing and sterilizing the exterior of the entire device. In one embodiment, the exterior is sterilized using 70% ethanol so that the sterility may be maintained in any post-processing steps, such as in a laminar air flow bench setup, *e.g.,* for isolation of cell material according to the present invention, and for fluid storage.

In one embodiment, the amniotic fluid collection procedure is performed in less than one minute. In one embodiment, the amniotic fluid collection procedure is performed in one to two minutes. In one embodiment, the amniotic fluid collection procedure is performed in not more than three minutes. In one embodiment, the method is simplified compared to standard operating procedures for cesarean sections, for example, by preventing spillage of the amniotic fluid into the operating wound, improving visibility and physical access. In one embodiment, fetal skin is unaffected by the device tip.

### Isolation, culture, sub-culture, reprogramming and differentiation of cells derived from amniotic fluid

Also provided are methods for isolation of cells from amniotic fluid, such as late-stage or full-term amniotic fluid, *e.g.,* collected using the methods and apparatuses provided herein. In one embodiment, after external sterilization, the device is transferred to a laminar air flow bench, where the outlet from the collection chamber is opened for removal of amniotic fluid. A portion of the amniotic fluid can be retained for reference purposes.

In one embodiment, the amniotic fluid is filtered to remove debris, such as to separate the vernix and large tissue debris aggregates. A number of appropriate filtration methods are known and may be used in connection with this embodiment. In one example, fluid is filtered through a 100 micron nylon mesh. After filtration, cells are separated, for example, by centrifugation, such as differential centrifugation, for example, using a gradient, *e.g.,* density gradient, *e.g.,* sucrose gradient, or Ficoll-sucrose gradient. In one example, a Ficoll 400 sucrose gradient is used for centrifugation at room temperature for 5 minutes at 500 g. Following gradient centrifugation, the cell-containing layer can be collected, *e.g.,* with a pipette.

Cells can be counted to determine yield. In one embodiment, the procedure yields at least at or about 10%, 20%, 30%, 40%, 50%, 60%, 70%, 80%, 90% viable cells. In preferred embodiments, the procedure yields at least at or about 50% viable cells. In one embodiment, the method yields at least at or about 1, 2, 3, 4, 5, 6, 7, 8, 9, or 10 million viable cells per liter of amniotic fluid collected.

In some embodiments, the isolated cells are cultured, for example, to expand the cells. In one example, the cells are transferred to a culture receptacle, e.g., flask, containing a growth medium appropriate for the desired cell-type. Exemplary media are DMEM supplemented with Fetal Calf Serum (FCS), fibroblast/mesenchymal stem cell growth medium, endothelial cell growth medium (ECM), and small-airway epithelial growth medium (SAGM). In one embodiment, a fibroblast growth medium is used. In one embodiment, a mesenchymal stem cell growth medium is used. In one embodiment, an endothelial cell growth medium is used. In one embodiment, a small-airway epithelial growth medium is used. Without being bound by any theory, other cell culture medium may be used in certain embodiments. In one aspect, the isolated cells are cultured in the medium for at least or about one week, two weeks, three weeks, four weeks, or five weeks. In certain aspect, the isolated cells are cultured in the medium for at least or about 11, 12, 13, 14, 15, 16, 17,18, 19, 20, 21, 22, 23, 24, 25, 26, 27, 28, 29 or 30 days. Flow cytometry, microscopy, and other known procedures may be used to assess the behavior and phenotype of the cultured cells.

In one embodiment, the cells are propagated, *e.g*., in sub-cultures. In one example, the cells are propagated for at least at or about 10, 20, 30, 40, 50, 60, or 70 days, or for 30 population-doublings, or until the cells have expanded at least 10,000-, 100,000-, 200,000-, 300,000-, 400,000-, 500,000-, or 1 million-, 2 million-, or 3 million-fold, or until the cells have reached senescence or until just before the cells reach senescence.

In one embodiment, high expression of Oct-4 is maintained by secondary culture in hypoxic conditions, corresponding to the low oxygen tension in-utero.

Also provided are methods for reprogramming and differentiating the cells, for example, to generate desired types of cells or tissues, such as for regenerative therapeutic applications. In certain embodiments, the cells are reprogrammed to a less-differentiated stage, such as to a pluripotent or multipotent stage. Reprogramming methods, such as induction of pluripotent stem cells (iPSC) are well-known and may be used to revert the differentiation of the cells to generate pluripotent or multipotent cells. Exemplary methods are described in Takahashi and Yamanaka, 2006, Cell 126(4): 663-676, and in Yu et al., 2007, Science 318(5858): 1917-1920.

In some embodiments, the cells, e.g., pluripotent or multipotent cells, are differentiated into desired cell- or tissue-types. In one aspect, the cells are cultured under conditions for generation of a desired cell type or tissue type, for example, endodermal, ectodermal, or mesodermal-derived tissues, such as liver cells or tissue, endocrine tissue, lung cells or tissue, blood cells, bone marrow cells, neuronal cells, astroglial cells, heart cells or tissue, e.g., cardiomyocytes, ocular cells or tissue, nerve cells or tissue, brain cells or tissue, muscle cells or tissue, skin cells or tissue, pancreatic cells or tissue, e.g., beta cells, adipogenic cells, chondrogenic cells, osteogenic cells, and neural cells. In certain embodiments, the cells are differentiated into neurons, hepatocytes, pancreatic islet cells, kidney cells, and hematopoietic cells, among other cell types. In some embodiments, the cells are differentiated into cell types representing all three germ layers. In some embodiments, the cells maintain an efficient differentiation capacity across germ layers following reprogramming. In one aspect, the cells possess a higher blood generation capacity compared to human embryonic stem cells or cell lines. One such human embryonic stem cell line is H9. In another aspect, the cells possess near 100% neuronal cell differentiation capacity. In some embodiments, the cells are differentiated to osteoblasts or adipocytes.

Differentiation methods are well-known. Any of a number of known methods may be used in connection with this embodiment, for example, the methods described in U.S. Patent No. 7,596,385, U.S. Patent Publication Numbers US 2005/0054093 and US 2005/0042595, all to Haas, and in U.S. Patent Publication Number US 2005/0124003 and International Patent Application No. WO 03/042405, to Atala et al., and methods described in "Teratocarcinomas and embryonic stem cells: A practical approach," E. J. Robertson, ed., IRL Press Ltd. 1987; "Guide to Techniques in Mouse Development," P. M. Wasserman et al. eds., Academic Press 1993; "Embryonic Stem Cell Differentiation in vitro, M. V. Wiles, Meth. Enzymol. 225: 900, 1993; "Properties and uses of Embryonic Stem Cells Prospects for Application to Human Biology and Gene Therapy," P. D. Rathjen et al., Reprod. Fertil. Dev. 10: 31, 1998; and in "Stem cell biology," L. M. Reid, Curr. Opinion Cell Biol. 2: 121, 1990.

Differentiation-inducing agents, maturation agents, and maturation factors also are well-known for differentiation to particular cell types, such as agents described in U.S. Pat. No. 6,506,574, to Rambhatla et al., e.g., N-butyrate, for differentiation into liver cells, and other maturation agents, maturation factors, growth factors, peptide hormones, cytokines, ligand receptor complexes, corticosteroids, retinoic acid, and organic solvents, such as DMSO, glucocorticoid with cAMP-elevating agents, methyl-isobutylxanthine, and indomethacin. Choice of differentiation agent(s) will depend on desired cell or tissue type.

While various embodiments of the invention have been described above, it should be understood that they have been presented by way of example only, and not by way of limitation. Likewise, the various diagrams may depict an example architectural or other configuration for the disclosure, which is done to aid in understanding the features and functionality that can be included in the disclosure. The disclosure is not restricted to the illustrated example architectures or configurations, but can be implemented using a variety of alternative architectures and configurations. Additionally, although the disclosure is described above in terms of various exemplary embodiments and implementations, it should be understood that the various features and functionality described in one or more of the individual embodiments are not limited in their applicability to the particular embodiment with which they are described. They instead can, be applied, alone or in some combination, to one or more of the other embodiments of the disclosure, whether or not such embodiments are described, and whether or not such features are presented as being a part of a described embodiment. Thus the breadth and scope of the present disclosure should not be limited by any of the above-described exemplary embodiments.

### D. Fetal and infant cells derived from amniotic fluid

Also provided are cells isolated from amniotic fluid, such as fetal and infant cells. Typically, the cells are present at abundant quantities. In one example, the cells are capable of culture, expansion in cell culture, reprogramming, differentiation, and/or clonal selection. In another example, the cells are adherent. In another example, the cells contain intact genomes, for example, with fewer mutations than a cell obtained from adult sources, such as a mesenchymal stem cell.

In one aspect, the cells express very few or very low levels of or do not express hematopoietic markers, such as CD45. In another aspect, the cells have surface expression of progenitor markers, such as CD90 and CD34. In another aspect, the cells have surface expression of CD73. In some embodiments, about 99 % of the cells in a population are positive for surface expression of CD90, about 0 % of the cells in a population are positive for surface expression of CD45, about 98 % of the cells in a population are positive for surface expression of CD73, and about 14 % of the cells in a population are positive for surface expression of CD34. In some examples, at least at or about 15 %, 20 %, 25 %, 30 %, 35 %, 40 %, 45 %, 50 %, 55 %, 60 %, 65 %, 70 %, 75 %, 80 %, 85 %, 90 %, 95 %, 96 %, 97 %, 98 %, 99 % or 100 % of the cells in a population are positive for surface expression of CD90, CD 34, and/or CD73. In another aspect, the cells are negative for surface expression of CD105 or express CD105 at low levels. In one example, only a very small percentage, such as less than at or about 10, 5, 4, 3, 2, 1, 0.5 %, or fewer of the cells, in a population are positive for surface expression of CD105 and/or CD45 or other hematopoietic markers.

In one example, the cells exhibit fibroblast-like morphology and growth characteristics, for example, having similar shape, size, and adherent properties as fibroblasts when observed using light microscopy. In another example, the cells are epithelial mesenchymal in origin.

In one example, the cells have a high proliferative capacity, such as the ability to grow in continuous culture for at least 10, 20, 30, 40, 50, 60, 70, or more days, the ability of proliferating in culture for at least 30 population doublings, or the ability to expand at least 10,000-, 100,000-, 200,000-, 300,000-, 400,000-, 500,000-, or 1 million-, 2 million-, or 3 million-fold in culture. In some examples, the cells are mortal, *i.e.,* do not survive indefinitely in culture. In some examples, the cells are multipotent or pluripotent, or capable of reprogramming into a multipotent or pluripotent cell type.

### E. Uses of the cells

The cells provided herein are useful in a variety of therapeutic and other applications, such as therapeutics, treatment, disease prevention, drug discovery, personalized medicine, regenerative medicine, tissue and cell generation, and universal donor banks. The cells are suitable for tissue engineering purposes, transplantation, therapeutic molecule production in-vivo or in-vitro, in both personalized medicine applications, as well as in universal donor banks. Thus, also provided are methods for use of the provided cells and compositions in such therapeutic and other methods.

In some embodiments, the fluid and/or cells provided herein are cryopreserved, for example, in a cryoprotective solution comprising a medium or buffer and a cryoprotective agent, for example, for storage in a cryogenic biobank. In some aspects, the cells are subject to pre-storage and post storage manipulation.

The cells may be cryopreserved using standard techniques. Examples of media for use in cryopreservation are Dulbecco's Modified Eagle Medium (DMEM), Medium 199 (M199), F-12 Medium, and RPMI Medium. An example of a buffer is phosphate buffered saline (PBS). Examples of cryoprotective agents are dimethylsulfoxide (DMSO) and glycerol. Examples of cryoprotective solutions are: DMEM/glycerol (1:1), DMEM/7.5% DMSO, M199/7.5% DMSO, and PBS/3.5 M DMSO. Optionally, the samples may be treated with antibiotics such as penicillin or streptomycin prior to cryopreservation. Cryopreservation may be accomplished using a rapid, flash-freeze method or by more conventional controlled rate-freeze methods. Rapid freezing of amniotic tissue may be accomplished by placing sample(s) in a freezing tube containing a cryoprotective solution and then rapidly immersing the freezing tube in liquid nitrogen. General slow freezing may be accomplished by placing sample(s) in a freezing tube containing a cryoprotective solution and then placing the freezing tube in a -70° C. freezer. Alternatively, the sample(s) may be subjected to controlled rate freezing using a standard cryogenic rate controlled system. Products of the cells may be used in reconstructive treatment, either in vivo or ex vivo, and to produce factors such as growth factors, cytokines, and other biological response modifiers.

In some embodiments, the cells are used in genetic modification methods, such as in transfection procedures to introduce a gene of interest using well-known genetic modification techniques, for example, to introduce a gene to improve the viability of the cells or render the cells immortal, such as by introduction of a TERT (telomerase reverse transcriptaste) gene.

The cells, compositions, and tissues provided herein, such as differentiated cells, are useful for the treatment of diseases, such as, but not limited to, infertility, cirrhosis of the liver, pancreatitis, diabetes, Parkinson's disease, spinal cord injury, stroke, burns, heart disease, osteoarthritis, rheumatoid arthritis, cancers, such as leukemia, e.g., Acute Lymphoblastic Leukemia, Acute Myelogenous Leukemia, Acute Biphenotypic Leukemia, and Acute Undifferentiated Leukemia; Chronic Myelogenous Leukemia, Chronic Lymphocytic Leukemia, Juvenile Chronic Myelogenous Leukemia, Juvenile Myelomonocytic Leukemia, lymphoma, e.g., Non-Hodgkin's Lymphoma, Hodgkin's Disease, Multiple Myeloma, Plasma Cell Leukemia, Breast Cancer, Ewing Sarcoma, Neuroblastoma, Renal Cell Carcinoma, genetic blood disorders, brain disorders such as Alzheimer's disease, Refractory Anemia, Refractory Anemia with Ringed Sideroblasts, Refractory Anemia with Excess Blasts, Refractory Anemia with Excess Blasts in Transformation, Aplastic Anemia, Fanconi Anemia, Paroxysmal Nocturnal Hemoglobinuria, Pure Red Cell Aplasia, Acute Myelofibrosis, Agnogenic Myeloid Metaplasia, myelofibrosis, Polycythemia Vera, Essential Thrombocythemia, Chediak-Higashi Syndrome, Chronic Granulomatous Disease, Neutrophil Actin Deficiency, Reticular Dysgenesis, Mucopolysaccharidoses, Hurler's Syndrome, Scheie Syndrome, Hunter's Syndrome, Sanfilippo Syndrome, Morquio Syndrome, Maroteaux-Lamy Syndrome, Sly Syndrome, Beta-Glucuronidase Deficiency, Adrenoleukodystrophy, Mucolipidosis II, Krabbe Disease, Gaucher's Disease, Niemann-Pick Disease, Wolman Disease, Metachromatic Leukodystrophy, Familial Erythrophagocytic Lymphohistiocytosis, Histiocytosis-X, Hemophagocytosis, Inherited Erythrocyte Abnormalities, Beta Thalassemia Major, Sickle Cell Disease, Inherited Immune System Disorders, Ataxia-Telangiectasia, Kostmann Syndrome, Leukocyte Adhesion Deficiency, DiGeorge Syndrome, Bare Lymphocyte Syndrome, Omenn's Syndrome, Severe Combined Immunodeficiency, Common Variable Immunodeficiency, Wiskott-Aldrich Syndrome, X-Linked Lymphoproliferative Disorder, Other Inherited Disorders, Lesch-Nyhan Syndrome, Cartilage-Hair Hypoplasia, Glanzmann Thrombasthenia, Osteopetrosis, Inherited Platelet Abnormalities, Amegakaryocytosis, Congenital Thrombocytopenia, Plasma Cell Disorders, and Waldenstrom's Macroglobulinemia.

In certain embodiments, the cells are used in autologous/heterologous tissue regeneration/replacement therapy, including treatment of corneal epithelial defects, cartilage repair, facial dermabrasion, burn and wound dressing for traumatic injuries of skin, mucosal membranes, tympanic membranes, intestinal linings, and neurological structures. For example, augmentation of myocardial performance can be achieved by the transplantation of cells into damaged myocardium (cellular cardiomyoplasty (CCM)), to enhance myocardial performance and treat end-stage cardiac disease. The cells may also be used for repair of a number of CNS disorders, for example, as described by Cao et al., 2002, "Stem cell repair of central nervous system injury," J Neuroscience Res 68: 501-510. The cells may also be used in reconstructive treatment of damaged tissue by surgical implantation of cell sheets, disaggregated cells, and cells embedded in carriers for regeneration of tissues for which differentiated cells have been produced. The cells may also be used in tissue engineered constructs. Such constructs comprise a biocompatible polymer formed into a scaffold suitable for cell growth. The scaffold can be shaped into a heat valve, vessel (tubular), planar construct or any other suitable shape. Such constructs are well known, such as those described in WO02/035992, and in U.S. Pat. Nos. 6,479,064, 6,461,628.

It will be appreciated that, for clarity purposes, the above description has described embodiments of the invention with reference to different functional units and/or modules of the cell collection device. However, it will be apparent that any suitable distribution of functionality between different functional units, modules or domains may be used without detracting from the invention. For example, functionality illustrated to be performed by separate modules may be performed by the same module. Hence, references to specific functional units are only to be seen as references to suitable means for providing the described functionality, rather than indicative of a strict logical or physical structure or organization.

Terms and phrases used in this document, and variations thereof, unless otherwise expressly stated, should be construed as open ended as opposed to limiting. As examples of the foregoing: the term "including" should be read as meaning "including, without limitation" or the like; the term "example" is used to provide exemplary instances of the item in discussion, not an exhaustive or limiting list thereof; and adjectives such as "conventional," "traditional," "normal," "standard," "known", and terms of similar meaning, should not be construed as limiting the item described to a given time period, or to an item available as of a given time. But instead these terms should be read to encompass conventional, traditional, normal, or standard technologies that may be available, known now, or at any time in the future. Likewise, a group of items linked with the conjunction "and" should not be read as requiring that each and every one of those items be present in the grouping, but rather should be read as "and/or" unless expressly stated otherwise. Similarly, a group of items linked with the conjunction "or" should not be read as requiring mutual exclusivity among that group, but rather should also be read as "and/or" unless expressly stated otherwise. Furthermore, although items, elements or components of the disclosure may be described or claimed in the singular, the plural is contemplated to be within the scope thereof unless limitation to the singular is explicitly stated. For example, "a" device includes one or more devices. The presence of broadening words and phrases such as "one or more," "at least," "but not limited to", or other like phrases in some instances shall not be read to mean that the narrower case is intended or required in instances where such broadening phrases may be absent.

### EXAMPLES

The following examples are intended to further describe and illustrate various aspects of the invention, but not to limit, the scope of the invention in any manner, shape, or form, either explicitly or implicitly.

### EXAMPLE 1: Isolation of amniotic fluid cells

Amniotic fluid was collected from a healthy pregnant women at full term caesarean delivery for an uncomplicated cause (breech presentation, previous caesarean delivery, or fear of giving birth), after informed consent. Directly after incision of the uterine wall by a scalpel, the tip of the device described herein was inserted to about a 10 cm depth through the fetal membranes, and amniotic fluid was collected. In this example, the yield was a total of about 1200 ml. The inlet/hose to the collecting bag was closed and the device removed. No blood was visible in the material/amniotic fluid, which was a turbid fluid, which had a white/yellow color tinge. The collection procedure took less than a minute. The device tip did not experience clogging by vernix. Moreover, the operating staff found the operating procedure to be simplified compared to the standard operating procedures for cesarean sections, as the amniotic fluid (up to 1.5 liters) did not spill out into the operating wound, making visibility and physical access superior. Moreover, the fetal skin was unaffected by the device tip.

The device containing the collected material was put into a cooling box and immediately transferred to a cell-culture lab where the device was externally sterilized with 70% ethanol. In a laminar air flow bench, the outlet from the collection chamber was opened, and 320 ml of the amniotic fluid was used for isolating amniotic fluid cell material, with the remaining aliquot being used for chemical analysis and also stored for reference purposes. The amniotic fluid was filtered through a 100 micron nylon mesh to separate the vernix and larger tissue debris aggregates. The filtered material was then put onto a Ficoll 400 sucrose gradient and centrifuged at room temperature for 5 minutes at 500 g. The turbid, cell containing layer between the sucrose and water fraction, was collected with a pipette. A small drop of this fraction was deposited onto a Bürker-chamber (for cell counting) and examined in a light microscope. Trypan-blue was added to assess cell viability, and when counting nuclear containing cells with an intact external morphology, the typical percentage of viable cells at 2 hours after the collection procedure was 70%. The total viable cell count retrieved from the 400 ml was determined to be 2 million cells, i.e. 5 million cells per liter. The gradient centrifuged cell material was transferred to 25 cm² cell Falcon culture flasks containing a growth medium specific to the desired cell type being propagated. The collected cells were found to multiply in cell culture. FACS performed on the freshly collected cell fraction showed no signs of haematopoietic type cells, but high numbers of cells of epithelial and mesenchymal origin were observed.

### EXAMPLE 2: Isolation of an amniotic fluid surfactant fraction that retains its de-novo conformational state

Amniotic fluid was collected from a healthy pregnant woman at full term caesarean delivery for an uncomplicated cause (breech presentation, previous caesarean delivery, or fear of giving birth), after informed consent. Directly after incision of the uterine wall, the tip of the device described herein was inserted for about 10 cm through the fetal membranes, and amniotic fluid was collected. In this example, the yield was a total of 450 ml. The inlet/hose to the collecting bag was closed and the device removed. No blood was visible in the material/amniotic fluid, which was a turbid fluid, which had a white/yellow color tinge. The procedure of collecting the material was in all respects identical to Example 1.

The amniotic fluid sample was stored inside of the device collection chamber in refrigerator at 4°C until processing.

The amniotic fluid fraction was produced by the following steps:
1. Centrifugation step for removal of cells and cellular debris at 500 g for 5 minutes at 10°C using a Sigma 3-18K.
2. Retrieval of the cell and cell debris free amniotic fluid supernatant. Also removal of free floating sebum fat aggregates (originating from the fetal skin) by pipetting using a plastic pipette such as a Finnpipette 250 microliter universal tip.
3. Ultracentrifugation step at 15000 g at 10 degrees Celsius for ten minutes of supernatant from step 2 using a Sigma 3-18K.
4. Supernatant from step 3 was removed.
5. Surfactant pellet produced by step 3 was collected (with this pellet, small amounts of amniotic fluid are also included, keeping the pellet from drying).
6. This viscous pellet material collected in step 5 was subjected to yet another centrifugation step at 19000 g for one minute at 10 degrees Celsius in a Sigma microfuge.
7. The surfactant surface phase pellet is then stored in an Eppendorf 1.5ml tube with a small amount of excess amniotic fluid to protect from drying.
8. Should the surfactant pellet in step 7 appear too viscous, re-suspension with desired amount of the supernatant from step 3 is optionally performed.
9. The obtained surfactant fraction was divided into two aliquots of 200 microliters each. One was a control, and the other was subjected to air-exposure, where medical-grade air (Lund University Childrens Hospital central medical air supply) was bubbled through the material at a rate of 0.75 litres a minute for 5 minutes.

The control and air-exposed surfactant were analyzed by cryo-TEM, an artifact-free method well suited to study the lung surfactant. The sample of lung surfactant material prepared according to example 2, was spread on a carbon cryo-grid in minute amounts (5µl) using a pipette and thereafter vitrified by immediately being plunged into liquid ethane (-180°C). The samples were vitrified at 37°C and 100% humidity. The samples were viewed in a Philips Bio-twin 120 cryo with a La2B6 filament. The cryo-TEM samples were at all times kept below -180°C. Images were recorded using a Gatan CCD with Digital Micrograph 3.31 software for Macintosh. The air-exposed material showed significant structural alterations in the lipid-bilayer packing order, which seemed to be due to consumption of surfactant material from large, tightly packed lamellar bodies to tubular myelin like figures. When lung surfactant is excreted from type II cells, it is in the form of LB's, which then undergo a transformation into tubular myelin at the air-water interface. Thus, the primary structure for de-novo synthesized surfactant is retained by the device described herein. The most remarkable phenomenon was the increase of the volume of the surfactant fraction (corresponding to unpacking of primary packed surfactant into a structure incorporating more water). The aliquot exposed to air would increase its volume more than threefold (700 microliters).

### EXAMPLE 3: Isolation of cells from full-term amniotic fluid.

Amniotic fluid was collected from a healthy pregnant woman during full term caesarean delivery, which was performed for an uncomplicated cause after informed consent. In this example, the total yield of amniotic fluid was 450 mL. A prototype of the device described in Example 1 was used. No blood was visible in the collected amniotic fluid, which was turbid and had a white/yellow color tinge. The collection procedure took less than a minute. The collected material was put into a cooling box and immediately transferred to a cell-culture lab where the device was externally sterilized with 70% ethanol.

In a laminar air flow bench, the outlet from the collection chamber was opened, and 400 mL of the amniotic fluid was used for isolating cell material. The remaining volume of the collected fluid was used for chemical analysis and then stored for reference purposes. For isolation of cells, the 400 mL amniotic fluid was filtered through a 100 micron nylon mesh to separate the vernix and larger tissue debris aggregates. The filtered material was then put onto a Ficoll 400 sucrose gradient and centrifuged at room temperature for 5 minutes at 500 g. The cell-containing layer present in the interphase between the sucrose and water fractions then was collected with a pipette. For cell counting, a small drop of this fraction was deposited onto a Bürker-chamber and examined using a light microscope. Trypan-blue was added to assess cell viability and cells counted with an intact external morphology. The percentage of viable cells at 2 hours after the collection procedure was 70%. The total number of viable cells retrieved from the 450 mL amniotic fluid was determined to be 2 million cells (5 million cells per liter collected amniotic fluid).

The gradient centrifuged cell material was transferred to 25 cm² cell culture flasks containing a fibroblast growth medium. After five days in culture, the cells were observed by low resolution optical microscopy to confirm that the cells had multiplied in culture. The cells were adherent and had a fibroblastic morphology.

The cells were analyzed by FACS using known methods, to evaluate the surface expression of hematopoietic markers such as CD45, and of CD34, CD105, CD90, and CD73. Cells were stained with labeled antibodies (anti-CD90-APC, anti-CD73-PE, anti-CD 105-FITC, anti-CD45-FITC, and anti-CD34-APC), and the surface expression of the markers analyzed by flow cytometry. IgG with appropriate fluorochrome was used as a negative control. The results are shown in Table 1, below.

**Table 1 Expression of Cell Surface Markers**

| **marker** | **% cells positive** | **Overall Surface Phenotype** |
|---|---|---|
| CD90 | 99.8 | + |
| CD73 | 99.9 | + |
| CD105 | 2.69 | - |
| CD45 | 0.19 | - |
| CD34 | 14 | + |

As shown in Table 1, a high percentage of the cells was determined to have surface expression of CD90 (99.8 %), CD73 (99.9 %), and CD34 (14 %), while a very low percentage of the cells showed surface expression of CD105 (2.69 %) or CD45 (0.19 %). Thus, in this example, a population of fetal/infant cells was isolated from amniotic fluid that was substantially negative for hematopoietic surface markers such as CD45, positive for progenitor markers CD90 and CD34, positive for CD73, and substantially negative for CD105.

### EXAMPLE 4: Sub-Culture and Expansion of the Cells Derived From Amniotic Fluid.

Amniotic fluid was collected from a healthy pregnant woman at full term caesarean delivery for an uncomplicated cause after informed consent as described in Example 3. In this example, the total yield of amniotic fluid was 600 mL. Material was collected and cells isolated as described in Example 3.

The cells were sub-cultured and propagated in culture using the same conditions as in Example 3 for approximately 60 days. The cells expanded several 1000000-fold, reaching senescence at about 70 days. The number of population doublings was about 1 per day.

### EXAMPLE 5: Reprogramming of Amniotic Fluid-Derived Cells to a Pluripotent State and Differentiation into Cardiomyocytes

Amniotic fluid-derived fetal/infant cells were isolated, sub-cultured and expanded as described in Example 4. The cells were subjected to an induction of pluripotent stem cells (iPSC) procedure to revert their differentiation as in Takahashi and Yamanaka, 2006, Cell 126(4): 663-676. This procedure successfully dedifferentiated the cells, forming pluripotent stem cells.

The pluripotent cells then were differentiated in mesoderm-specifying medium, for generation of cardiomyocytes. In short, a serum-containing media with BMP-4 to drive mesoderm differentiation is sufficient. This procedure successfully differentiated the cells into beating cardiomyocytes, confirmed by live microscopy.

### EXAMPLE 6: Isolation of cells from full-term amniotic fluid.

In this example, large volumes of 1200 mL and 450 mL of amniotic fluid were obtained from two independent deliveries of normal healthy babies performed via caesarian section, using a specifically designed catheter based device. A prototype of the device described in Example 1 was used. In brief, the extracted fluid was obtained by the puncturing the amniotic membrane using a specially designed soft plastic piercing tip and collecting the fluid in a sterile bag via gravity flow syphoning. During the extraction, the fluid and its contents including cellular material from the fetus and the extra embryonic tissues were separated from the large particulate matter such as, the vernix. The extraction of the fluid was performed just prior to the infant's delivery. Once the fluid was harvested, cellular material was then isolated by filtration and density gradient centrifugation. Filtered samples were centrifuged at 850g for 5 min to pellet the cells down. Supernatant was removed and the pellet was resuspended in DMEM+10% FCS. Further separation was done by density gradient centrifugation of sample over lymphoprep (Medinor AB or AXIS-SHIELD) at 850g for 20 min. The isolated cells were counted by Trypan blue (Sigma-Aldrich), and plated on rat tail collagen I (BD Bioscience) pre-coated 6-well plates in either fibroblast cell medium (ScienCell Research laboratories), endothelial cell medium (ScienCell Research laboratories), small airway epithelial cell growth medium (Lonza), or DMEM+5% FCS. At day 11-25, emerged colonies in these media were either picked separately and expanded, or were pooled and expanded. The cells were split at a ratio of 1:5 or 1:7 every second or third day respectively. The supernatant was used to isolate surfactant and other bioactive molecules present via ultracentrifugation.

From the 480 and 250 mL fluid harvests, 11× 10⁶ and 6×10⁶ viable cells were obtained respectively.

### EXAMPLE 7: Sub-Culture and Expansion of the Cells Derived From Amniotic Fluid.

Cell harvested from the full-term amniotic fluid in Example 6 were plated into 4 kinds of culture media each containing: 1) fibroblast/mesenchymal stem cell growth (FCM); 2) endothelial cell growth (ECM); 3) small airway epithelial cell growth (SAGM); and 4) a generic DMEM based medium containing 5% fetal calf serum. Cell adhesion and proliferation, with the emergence of cell colonies occurred in the culture containing FCM, ECM, or SAGM. In these cultures, three unique types of cell colonies could be distinguished based on their morphology. Cultures containing FCM have a higher proportion of type 1 colonies, cultures containing ECM have a higher proportion of type 2 colonies, and cultures containing SAGM have a higher proportion of type 3 colonies. Continued culture of the cells revealed that the colonies with type 1 or type 2 morphology proliferated to form large colony sizes and reached plate-confluency within 11 days post-plating. The type 3 colonies showed limited growth potential and reached senescence within 24 days of culture, while no senescence was reached up to 60 days in culture for the type 1 and 2 colonies. Individual colonies of type 1, and 2 were picked and propagated separately in either FCM or ECM and both cell types were found to have doubling rates of approximately 20 hours. When amniotic fluid cells propagated without prior clonal selection or purification, following several passages using either the FCM or ECM media, the emerging adherent layer showed morphologically uniform cells of type 1 or type 2, corresponding to the cellular medium used. Therefore, the reprogramming experiments in Example 9 were performed on cellular material cultured without clonal selection.

### EXAMPLE 8: Characterization of Amniotic Fluid Derived Stem Cells.

Type 1 and type 2 cells (expanded in FCM and ECM, respectively) obtained in Example 7 were analyzed by flow cytometry for markers of fibroblasts, mesenchymal stem cells, endothelial cells, hematopoietic cells as well as pluripotency marker, OCT4. Flow cytometric analysis was performed as follows. Single-cell suspensions from confluent culture were prepared by gentle trypsinization. Cells were suspended in PBS+2% FCS and incubated with fluorescence-conjugated antibodies for 30 min. The antibodies used were CD45 (FitC-conjugated), CD34 (APC-conjugated), CD90 (APC-conjugated), CD73 (PE-conjugated), CD105 (FitC-conjugated) (Pharmingen). Isotype identical antibodies served as control to exclude nonspecific binding. Quantitative analysis was performed using FACSCanto flowcytometer (BECKMAN) and FlowJo software.

Both type 1 and type 2 cell colonies showed an identical cell surface phenotype despite a unique morphological appearance. See Figure 2 for FACS. The cells were negative for pan-hematopoietic cell marker CD45, were all CD90 and CD73 bright, and expressed low levels of CD105. A sub-fraction of cells were also positive for CD34. These cells express markers similar to those seen in previous studies of pre-term amniotic fluid derived stem cells from amniocentesis procedures with a mesenchymal stem cell phenotype. However, the full-term derived cells show a significantly reduced expression of CD105 compared to those studies. The cells also expressed the pluripotency marker Oct4 when first harvested, which was subsequently lost following culturing.

To further investigate the characteristics of type 1 and type 2 cells obtained from full-term amniotic fluid with mesenchymal stem cell phenotype, the differentiation potential of these cells into osteoblasts and adipocytes was tested using the standard 21-day osteogenic or adipogenic induction protocols. The multipotency of these cells was confirmed by detection of calcium deposit following Alizarin red staining, and lipid vacuoles accumulation following Oil red O staining.

For *in vitro* differentiation to osteoblasts, type 1 or type 2 cells were seeded at a density of 3×10³/cm² in complete NH expansion medium (Miltenyi) and allowed to adhere overnight. The next day, medium was changed to osteoblast induction medium (complete NH expansion medium+ 10mM β-glycerophosphate + 0.05 mM L-ascorbic acid-2-phosphate + 0.1 µM dexamethasone stock solution + 1% AB/AM solution (Sigma)). Cells were differentiated for 21 days while replacing the medium every 2-3 days. On day 21 cells were stained with Alizarin Red to measure calcium mineral content.

For *in vitro* differentiation to adipocytes, type 1 or type 2 cells were seeded at a density of 2×10⁴/cm² in complete NH expansion medium (Miltenyi) and cultured for 3-7 days, until cultures are 100% confluent. At day 7, medium was changed to NH AdipDiff medium (Miltenyi) + 1% AB/AM solution (Sigma). Cells were differentiated for 14 days while replacing the medium every 2-3 days. On day 14 cells were stained with Oil Red O to detect lipid vacuoles.

Together these results demonstrate that the most numerous and proliferative adherent cell type present in full-term amniotic fluid harvests is a unique full-term amniotic fluid derived stem cell (AFSC).

### EXAMPLE 9: Reprogramming of Full-Term Amniotic Fluid Stem Cell

The amniotic fluid derived stem cell (AFSC) obtained in Example 7 and characterized in Example 8 was evaluated for its ability to be reprogrammed. The adherent monolayer of cells was transduced with a lentiviral vector expressing the following reprogramming factors under the control of a tetracycline (tet) inducible promoter: OCT4 SOX2, LIN28, KLF4, and C-MYC. GFP expression was also under control of the tetracycline inducible promoter.

Specifically, lentiviral vectors FU-tet-o-hOct4, FU-tet-o-hSox2, FU-tet-o-hKlf4, FU-tet-o-hcMyc, FU-tet-o-hLin28, tet-inducible GFP marker vector, and FUdeltaGW-rtTA were independently used. To generate virus, 293T cells were transfected at 50-70% confluence. For a 10 cm plate, 10 ml of DMEM supplemented with 10% FCS and 1%P/S, and 40 µg DNA (10:7:3 vector:ΔR8.91 :vsv-g) were used. Medium was changed the next day to 6ml fresh medium. Virus was harvested the following 2 days and filtered using a 0.45µm filter. AFSCs were transduced with non-concentrated lentiviruses in 8-12 transduction cycles, to achieve a 60-80% transduction efficiency. Upon transducion 1ug/ml Dox was added to the medium of the control well to assess the transduction efficiency. Once reaching 80% transduction efficiency, cells were trypsinized and plated on murine embryonic fibroblast feeder cells in FCM or ECM+ 1ug/ml Dox. The culture medium of transduced cells was changed to 50% hESC medium+ 1ug/ml Dox 48h post-plating, and to 100% hESC+ 1µg/ml Dox medium 72h post-plating. The hESC medium+ 1ug/ml Dox was changed every second day until iPS-like colonies appeared. The iPS-like colonies (with sharp borders and packed cells with big nucleous) were picked at around day 20 to day 30 after viral transduction.

Following transduction of the cells, tetracycline was added to the media of control well to assess the percentage of GFP as a measure of the activation of the reprogramming factors. The highest reprogramming efficiency was obtained when a transduction efficiency of about 60-80% GFP was seen in the plated amniotic fluid stem cell population. In total, 42 iPS colonies were picked from the estimated 150,000 starting cells, yielding an iPS generation efficiency of approximately 0.03%. From these 42 iPS colonies, 9 were randomly selected for expansion and detailed characterization. All of the iPS clones tested for genomic integrity showed normal karyotype and had proliferation, morphological, and growth characteristics similar to those of human ES cell lines. All of the iPS cell lines were demonstrated to be pluripotent as assayed via real time PCR for the endogenous expression of pluripotency markers OCT4, SOX2, K1LF4, C-MYC, NANOG, DNMT3B, and TDGF1 (CRIPTO), with levels comparable to the human ES lines HI, HUES-3, and HUES-6. Seven of the 9 lines transplanted subcutaneously in Nod/Scid/I12rg-/- mice developed terato carcinomas. Five of the 7 terato carcinomas showed cells typical of the 3 germ layers, indicating the generation of mesoderm, endoderm, and ectoderm lineages. Two lines showed endoderm and mesodermal cell lineages. These results demonstrate feasibility of efficient generation of pluripotent stem cell lines from full-term AFSCs.

### EXAMPLE 10: Differentiation Potential of Full-Term AFSC Derived iPS Cell Lines

The AFSC derived iPS cell lines obtained in Example 9 were evaluated for their differentiation potential across multiple germ layers. The cell lines were differentiated using two well established high efficiency protocols for neural lineage differentiation and hematopoietic differentiation, respectively (Kirkeby et al., 2012, Cell reports 1(6): 603-614; Woods et al., 2011, Stem Cells 29(7): 1158-1164). A cord blood endothelial cell derived iPS cell line that has demonstrated high blood cell differentiation efficiency, and the human ES cell line, H9, which has been demonstrated to efficiently differentiate to neuronal lineages, were used as controls. Following differentiation of 3 randomly selected AFSC derived iPS cell lines, efficient differentiation to the blood (mesoderm) lineage, at levels significantly higher than the H9 ES cell line (22.2 +/- 4.4 % compared to 0.33% CD45+ cells from the H9 cell line) was observed. The cord blood derived iPS cell line showed high blood differentiation potential (50.9 +/- 5.1 % CD45+ cells; n=3). However, in contrast to very efficient differentiation of the AFSC-iPS cell lines towards neuronal lineages, the cord blood-iPS cell line continued to have cells differentiating to the mesoderm lineage despite the use of the potent neural differentiation protocol. Both the cord blood and the AFSC-iPS cell lines differentiated using the neuronal cell protocol showed cells positive for MAP2 (mature neuronal marker) and TUJ1 (immature neuronal marker), while there were significant non-neuronal cells (mesodermal fibrobalsts stained with TE7) in cord blood derived iPS cell line representing a more heterogeneous differentiation capacity. Moreover, the cord blood derived iPS cell line also showed compromised erythrocyte differentiation ability compared to the amniotic fluid derived iPS cell lines whereas all the AFSC-iPS cell lines could generate erythrocytes in colony forming unit assays. The human ES cell line H9 showed efficient neuronal lineage differentiation capacity with severely reduced blood cell potential. Bock et al., 2011, Cell 144(3): 439-452. Both the cord blood and AFSC derived iPS cell lines, gave rise to hematopoietic cells and progenitors (CD34) with the cord blood line yielding higher blood cell amounts (AF-iPS cell lines and CB-iPS cell lines gave rise to 22.2 +/- 4.4 % and 50.9 +/- 5.1 %; n=3 CD45+ cells respectively).

Microarray analysis of the iPS cell lines and the control cord blood iPS cell line and human ES cell line was performed, and the gene expression profiles of these cells were compared with the starting cell material (AFSC). A correlation between the starting cell material and the AFSC derived iPS cell lines for stem cell markers was observed, suggesting maintenance of several stem cell genes throughout the reprogramming. This observation indicated maintenance of the AFSC multipotency in the iPS cell lines. Taken together these results indicate that AFSCs are an ideal starting material for deriving novel iPS cell lines, and suggest that epigenetic memory maintains their broad stem cell potential throughout reprogramming to generate iPS cell lines with a multi-germ layer differentiation capacity.

Throughout this application, various website data content, publications, patent applications and patents are referenced. (Websites are referenced by their Uniform Resource Locator, or URL, addresses on the World Wide Web.) The disclosure of each of these references is hereby incorporated by reference herein in its entirety.

The present invention is not to be limited in scope by the embodiments disclosed herein, which are intended as single illustrations of individual aspects of the invention, and any that are functionally equivalent are within the scope of the invention. Various modifications to the models and methods of the invention, in addition to those described herein, will become apparent to those skilled in the art from the foregoing description and teachings, and are similarly intended to fall within the scope of the invention. Such modifications or other embodiments can be practiced without departing from the true scope and spirit of the invention.

In an example of the above disclosure:
Example 1: An amniotic fluid collection device, comprising: a collection chamber; a collection chamber outlet valve coupled to the collection chamber; a collection chamber inlet valve coupled to the collection chamber; a proximal fluid-extraction tube coupled to the collection chamber inlet valve, the proximal fluid-extraction tube comprising a medical grade material; and a distal fluid-extraction tube coupled to the proximal fluid-extraction tube, the distal fluid extraction tube comprising a transfer portion and an inlet, wherein the inlet comprises a main inlet, a penetrating tip, and one or more lateral inlets.
Example 2: An isolated fetal or infant cell, comprising surface expression of a marker selected from the group consisting of CD73 and CD90, wherein: the surface of the cell is negative for CD105 expression; or the surface of the cell expresses CD34.
Example 3: The isolated cell of example 2, wherein the cell comprises surface expression of CD73 and CD90.
Example 4: The isolated cell of example 2 or example 3, wherein the cell surface is negative for CD105 expression and expresses CD34.
Example 5: The isolated cell of any of examples 2-4, wherein the cell expresses the transcription factor Oct-4.
Example 6: The isolated cell of any of examples 2-5, wherein the cell surface is negative for CD45 surface expression.
Example 7: The isolated cell of any of examples 2-6, wherein the cell is adherent.
Example 8: The isolated cell of any of examples 2-7, wherein the cell has a fibroblastic morphology.
Example 9: The isolated cell of any of examples 2-8, wherein the cell is pluripotent or multipotent.
Example 10: The isolated cell of any of examples 2-9, wherein: the cell is capable of growing in continuous culture for at least 50 days; or the cell is capable of proliferating in culture for at least 30 population doublings; or the cell is capable of expanding at least 1,000,000 fold in continuous culture.
Example 11: A composition, comprising a fetal or infant cell of any of examples 2-10.
Example 12: A composition, comprising a plurality of fetal or infant cells, wherein at least 10 % of the cells have surface expression of CD90, CD73, and CD34 and less than 5 % of the cells have surface expression of CD105.
Example 13: The composition of example 12, wherein at least 50 % of the cells express the transcription factor Oct-4.
Example 14: The composition of example 12 or example 13, wherein less than 5 % of the cells have CD45 surface expression.
Example 15: The composition of any of examples 12-14, wherein at least 50 % of the cells are adherent.
Example 16: The composition of any of examples 12-15, wherein at least 50 % of the cells have a fibroblastic morphology.
Example 17: The composition of any of examples 12-16, wherein at least 50 % of the cells are pluripotent or multipotent.
Example 18: The composition of any of examples 12-14, wherein at least 50 % of the cells are capable of growing in continuous culture for at least 50 days; or the plurality of cells is capable of proliferating in culture for at least 30 population doublings; or the plurality of cells is capable of expanding at least 1,000,000 fold in continuous culture.
Example 19: The composition of any of examples 11-18, further comprising amniotic fluid.
Example 20: The composition of example 19, wherein the cells are present at a concentration of at least 1 million cells per liter.
Example 21: A method for isolating fetal or infant cells, comprising: (a) obtaining amniotic fluid at a gestational stage of greater than 21, 22, 23, 24, 25, 26, 27, 28, 29, 30, 31, 32, 33, 34, 35, 36, 37, 38, or 39 weeks; and (b) recovering fetal or infant cells from the amniotic fluid.
Example 22: The method of example 21, wherein the obtaining in step (a) comprises collecting amniotic fluid by caesarean section.
Example 23: The method of example 21 or 22, wherein the recovering in step (b) comprises removing particulate matter from the amniotic fluid.
Example 24: The method of example 23, wherein the recovering in step (b) further comprises performing gradient centrifugation.
Example 25: The method of any of examples 21-24, further comprising culturing the recovered cells under conditions whereby the cells proliferate.
Example 26: The method of any of examples 21-25, wherein the recovered cells in step (b) comprise at least 1 million cells per liter of amniotic fluid obtained in step (a).
Example 27: The method of any of examples 21-26, wherein the amount of amniotic fluid obtained in step (a) is at least at or about 10 mL, 20 mL, 30 mL, 40 mL, 50 mL, 60 mL, 70 mL, 80 mL, 90 mL, 100 mL, 200 mL, 300 mL, 400 mL, 500 mL, 600 mL, 700 mL, 800 mL, 900 mL, or 1000 mL.
Example 28: The method of any of examples 21-27, wherein the amniotic fluid is full-term amniotic fluid.
Example 29: The method of any of examples 21-28, wherein the fetal or infant cells comprise the cell of any of examples 2-10.
Example 30: The method of any of examples 21-28, wherein the fetal or infant cells comprise a cell that is positive for surface expression of a marker selected from the group consisting of CD73 and CD90, wherein the cell is negative for surface expression of CD105 expression or positive for surface expression of CD34.
Example 31: The method of example 30, wherein the cell is positive for surface expression of CD73 and CD90.
Example 32: The method of example 30 or 31, wherein the cell is negative for surface expression of CD105 and is positive for surface expression of CD34.
Example 33: The method of any of examples 30-32, wherein the cell expresses the transcription factor Oct-4.
Example 34: The method of any of examples 30-33, wherein the cell is negative for CD45 surface expression.
Example 35: The method of any of examples 30-34, wherein the cell is adherent.
Example 36: The method of any of examples 30-35, wherein the cell has a fibroblastic morphology.
Example 37: The method of any of examples 30-36, wherein the cell is pluripotent or multipotent.
Example 38: The method of any of examples 30-37, wherein: the cell is capable of growing in continuous culture for at least 50 days; or the cell is capable of proliferating in culture for at least 30 population doublings; or the cell is capable of expanding at least 1,000,000 fold in continuous culture.
Example 39: The method of any of examples 21-38, further comprising reprogramming the recovered cells, thereby generating multipotent or pluripotent cells.
Example 40: The method of example 39, wherein the reprogramming comprises performing iPSC.
Example 41: The method of any of examples 21-40, further comprising differentiating the cells into a desired cell or tissue type.
Example 42: The method of example 41, wherein the desired cell or tissue type is selected from the group consisting of hematopoietic cells, neuronal cells, endodermal cells, ectodermal cells, and mesodermal cells.
Example 43: A method of treatment, comprising administering the composition of any of examples 11-18 to a patient.
Example 44: A method for banking cells, comprising (a) obtaining one or more samples, each containing the cell of any of examples 2-10; (b) storing the sample under conditions to preserve viability of at least some of the cells in the sample; and (c) storing data related to the identity of the subject from which the sample was obtained.
Example 45: The method of example 44, wherein the storing comprises treating the sample with one or more cryoprotective agent.
Example 46: The method of example 44 or example 45, wherein step (a) comprises obtaining a plurality of samples, each obtained from a different subject.
Example 47: A cell bank, comprising (a) a plurality of samples, each containing the cell of any of examples 2-10 or the composition of any of examples 11-20; and (b) a database containing data for individual identification and retrieval of the samples.
Example 48: The cell bank of example 47, wherein each of the plurality of samples is derived from an individual subject.
Example 49: The cell bank of example 47 or 48, wherein the data includes information about the identity of the subjects from which individual samples were derived.
Example 50: A method for collecting amniotic fluid, comprising: (a) inserting an amniotic fluid collection device through an incision in the uterine wall of the subject; (b) penetrating the amniotic membrane of the subject; and (c) collecting amniotic fluid from the amniotic sac of the subject, wherein the subject is a pregnant mother at a stage of at least 30 gestational weeks.
Example 51: The method of example 50, wherein the subject is at a full-term stage of gestation.
Example 52: The method of example 50 or 51, wherein the amniotic fluid collection device is the device of example 1.
Example 53: The method of any of examples 50-52, wherein step (b) further comprises penetrating a chronic membrane.
Example 54: The method of any of examples 50-53, wherein the collecting in step (c) includes one or more of: (i) initiating a siphon to transfer the amniotic fluid to a collection chamber of the amniotic fluid collection device by opening an inlet valve of the amniotic fluid collection device; (ii) positioning a collection chamber of the amniotic fluid collection device below an inlet of the amniotic fluid collection device; (iii) coupling a negative pressure source to an outlet of the amniotic fluid collection device to initiate transfer of the amniotic fluid; and (iv) relocating an inlet of the amniotic fluid collection device to retrieve substantially all of the available amniotic fluid.
Example 55: The method of any of examples 50-54, further comprising step (d) removing the amniotic fluid collection device.
Example 56: The method of any of examples 50-55, wherein the incision is made by the amniotic fluid collection device.
Example 57: The method of any of examples 50-56, wherein the method is performed in less than about 10 minutes, about 9 minutes, about 8 minutes, about 7 minutes, about 6 minutes, about 5 minutes, about 4 minutes, about 3 minutes, about 2 minutes, or about 1 minute.

## Claims

1. A method for isolating fetal or infant cells, comprising:
(a) obtaining amniotic fluid at a gestational stage of greater than 21, 22, 23, 24, 25, 26, 27, 28, 29, 30, 31, 32, 33, 34, 35, 36, 37, 38, or 39 weeks; and
(b) recovering fetal or infant cells from the amniotic fluid.

2. The method of claim 1, wherein the recovering in step (b) comprises removing particulate matter from the amniotic fluid.

3. The method of claim 2, wherein the recovering in step (b) further comprises performing gradient centrifugation.

4. The method of any of claims 1-3, further comprising culturing the recovered cells under conditions whereby the cells proliferate.

5. The method of any of claims 1-4, wherein the recovered cells in step (b) comprise at least 1 million cells per liter of amniotic fluid obtained in step (a).

6. The method of any of claims 1-5, wherein the amount of amniotic fluid obtained in step (a) is at least at or about 10 mL, 20 mL, 30 mL, 40 mL, 50 mL, 60 mL, 70 mL, 80 mL, 90 mL, 100 mL, 200 mL, 300 mL, 400 mL, 500 mL, 600 mL, 700 mL, 800 mL, 900 mL, or 1000 mL.

7. The method of any of claims 1-6, wherein the amniotic fluid is full-term amniotic fluid.

8. The method of any of claims 1-7, wherein the fetal or infant cells comprise a cell that is positive for surface expression of a marker selected from the group consisting of CD73 and CD90, wherein the cell is negative for surface expression of CD105 expression or positive for surface expression of CD34.

9. The method of claim 8, wherein the cell is positive for surface expression of CD73 and CD90.

10. The method of any of claims 8-9, wherein the cell expresses the transcription factor Oct-4.

11. The method of any of claims 8-10, wherein the cell is negative for CD45 surface expression.

12. The method of any of claims 8-11, wherein the cell is adherent.

13. The method of any of claims 8-12, wherein the cell has a fibroblastic morphology.

14. The method of any of claims 8-13, wherein the cell is pluripotent or multi potent.

15. The method of any of claims 8-14, wherein:
the cell is capable of growing in continuous culture for at least 50 days; or
the cell is capable of proliferating in culture for at least 30 population doublings; or
the cell is capable of expanding at least 1,000,000 fold in continuous culture.
